# EUROPEAN PATENT APPLICATION

(11) **EP 4 438 296 A1**
(43) Date of publication of application: **02.10.2024**
(21) Application number: 22898402.7
(22) Date of filing: 09.11.2022
(51) Int. Cl.: B32B 27/32, B65D 65/40

(54) **LAMINATED FILM, CONTAINER, CELL CULTURE CONTAINER, CELL CULTURE METHOD AND METHOD FOR MANUFACTURING CELL CULTURE CONTAINER**

(30) Priority: 24.11.2021 JP 2021190482
(71) Applicant: Mitsui Chemicals, Inc., Tokyo 104-0028 (JP)
(72) Inventor: KATAYAMA, Yoshiyuki, Tokyo 104-0028 (JP)
(74) Representative: J A Kemp LLP
(86) International application number: PCT/JP2022/041741
(87) International publication number: WO 2023/095618

(57) **Abstract**

A laminated film includes a first polyolefin-based resin layer including a first polyolefin-based resin having a melting point of from 95°C to 200°C, a second polyolefin-based resin layer including a second polyolefin-based resin having a melting point of less than 95°C, and a third polyolefin-based resin layer including a third polyolefin-based resin having a melting point of from 95°C to 200°C, which are disposed in this order. The laminated film has an oxygen permeability of from 7,500 mL/m²·day·atm to 85,000 mL/m²·day·atm.

## Description

### Technical Field

The present disclosure relates to a laminated film, a container, a cell culture container, a cell culture method, and a method of producing a cell culture container.

### Background Art

Films in which low-density resins are used have been developed. By using low-density resins, films having excellent gas permeability can be obtained.

For example, 4-methyl-1-pentene polymers have bulky functional groups, and, therefore, have lower densities than those of other thermoplastic polyolefin laminated films. As a result, laminated films including 4-methyl-1-pentene polymers have high gas permeablity with respect to, for example, oxygen gas and carbon dioxide gas. Such laminated films can be used as gas-permeable laminated films for packaging materials for fresh foods or the like.

For example, Patent Document 1 discloses a bulb packaging bag obtained by molding a 4-methyl-1-pentene resin composition, which contains from 99 to 70 parts by mass of a 4-methyl-1-pentene polymer and from 1 to 30 parts by mass of a butene-1-based solid polymer, into a film such that the degree of crystallinity is in a range of from 25 to 35%, and subsequently shaping the film into a bag shape by means of a heat-sealing method.

Patent Document 1: Japanese Patent Application Laid-Open (JP-A) No. H11-301691

### SUMMARY OF THE INVENTION

### Technical Problem

As described above, from the standpoint of obtaining excellent gas permeability, it is considered that it is preferable to produce a film using a low-density resin (e.g., a low-density polyolefin-based resin).

However, low-density resins tend to have low melting points. In attempts to produce a film using a low-melting-point resin, there have been problems in that, for example, the resulting film is sticky at normal temperature, film production itself is not possible due to the resin being unable to maintain its shape, and the resulting film has poor heat sealability. In other words, it has been difficult to produce a film that retains product quality when a low-melting-point resin is used.

In view of the above, it is desired to produce a film having excellent gas permeability by using a low-melting-point resin.

A problem to be solved by one embodiment of the present disclosure is to provide a laminated film that includes a low-melting-point polyolefin-based resin and has excellent gas permeability, a container obtained from the laminated film, a cell culture container, a cell culture method, and a method of producing a cell culture container.

### Solution to Problem

Means for solving the above-described problem include the following aspects.
<1> A laminated film, including:
   a first polyolefin-based resin layer including a first polyolefin-based resin having a melting point of from 95°C to 200°C;
   a second polyolefin-based resin layer including a second polyolefin-based resin having a melting point of less than 95°C; and
   a third polyolefin-based resin layer including a third polyolefin-based resin having a melting point of from 95°C to 200°C,
   wherein the first polyolefin-based resin layer, the second polyolefin-based resin layer, and the third polyolefin-based resin layer are disposed in this order, and the laminated film has an oxygen permeability of from 7,500 mL/m²·day·atm to 85,000 mL/m²·day·atm.
<2> The laminated film according to <1>, wherein the laminated film has a total thickness of from 15 µm to less than 150 µm.
<3> The laminated film according to <1> or <2>, wherein the second polyolefin-based resin layer has a density of from 820 kg/m³ to less than 890 kg/m³ as measured in accordance with JIS K7112.
<4> The laminated film according to any one of <1> to <3>, wherein the second polyolefin-based resin is an ethylene-*α*-olefin copolymer having a melting point of less than 90°C.
<5> The laminated film according to any one of <1> to <4>, wherein each of the first polyolefin-based resin layer and the third polyolefin-based resin layer has a density of from 900 kg/m³ to 980 kg/m³ as measured in accordance with JIS K7112.
<6> The laminated film according to any one of <1> to <5>, wherein the laminated film has a carbon dioxide permeability of from 35,000 mL/m²·day·atm to 250,000 mL/m²·day·atm.
<7> The laminated film according to any one of <1> to <6>, wherein at least one of the first polyolefin-based resin layer or the third polyolefin-based resin layer has a thickness of from 5 µm to 100 µm.
<8> The laminated film according to any one of <1> to <7>, wherein the second polyolefin-based resin layer has a thickness of from 5 µm to 100 µm.
<9> The laminated film according to any one of <1> to <8>, wherein the ratio of the thickness of the second polyolefin-based resin layer to the total thickness of the first polyolefin-based resin layer and the third polyolefin-based resin layer is from 0.5 to 20.0.
<10> The laminated film according to any one of <1> to <9>, wherein the laminated film is formed by a coextrusion blown film molding method, a coextrusion cast film molding method, or a coextrusion lamination molding method.
<11> A container, formed from the laminated film according to any one of <1> to <10>, wherein the container is heat-sealed.
<12> A cell culture container, including the laminated film according to any one of <1> to <10>.
<13> The cell culture container according to <12>, including the laminated film at a bottom portion of the container.
<14> The cell culture container according to <12> or <13>, wherein edge portions of the laminated film are heat-sealed to form a bag.
<15> The cell culture container according to <14>, having a medical device Sterility Assurance Level (SAL) of 10⁻⁶ or less as measured in accordance with BS EN 556-1:2001.
<16> A cell culture method, including a step of enclosing a cell in the cell culture container according to any one of <12> to <15> and hermetically sealing the cell culture container.
<17> A method of producing a cell culture container, the method including a film-forming step of coextruding a third polyolefin-based resin layer including a first polyolefin-based resin having a melting point of from 95°C to 200°C, a second polyolefin-based resin layer including a second polyolefin-based resin having a melting point of less than 95°C, and a third polyolefin-based resin layer including a third polyolefin-based resin having a melting point of from 95°C to 200°C, to obtain a laminated film.
<18> The method of producing a cell culture container according to <17>, further including a bag-forming step of heat-sealing edge portions of the obtained laminated film, to form a bag.
<19> The method of producing a cell culture container according to <18>, further including a step of irradiating the obtained bag with a gamma ray.
<20> The method of producing a cell culture container according to any one of <17> to <19>, wherein, in the film-forming step, the laminated film is obtained using a blown film molding machine.

### Advantageous Effects of Invention

According to one embodiment of the present disclosure, a laminated film that includes a low-melting-point polyolefin-based resin and has excellent gas permeability, a container obtained from the laminated film, a cell culture container, a cell culture method, and a method of producing a cell culture container are provided.

### DESCRIPTION OF EMBODIMENTS

Specific embodiments of the present disclosure are described below in detail. However, the present disclosure is not limited to the below-described embodiments in any way, and the present disclosure can be carried out with modifications, as appropriate, within the scope of the purpose of the present disclosure.

In the present specification, any numerical range expressed using "to" denotes a range that includes the numerical values noted before and after "to" as the lower limit value and the upper limit value, respectively.

In the present specification, a "face" of a member means a "main face" of the member, unless specified otherwise.

In the present specification, "adhesion" indicates a notion that encompasses "pressure-sensitive adhesion".

In the present specification, "laminated film" indicates a concept that encompasses not only materials that are generally referred to as "films", but also materials that are generally referred to as "sheets".

In the present specification, in a case in which there are plural substances that correspond to a particular component in a composition, any description of the amount of the component in the composition means the total amount of the plural substances present in the composition, unless otherwise specified.

### <<Laminated Film>>

The laminated film of the present disclosure includes a first polyolefin-based resin layer that includes a first polyolefin-based resin having a melting point of from 95°C to 200°C, a second polyolefin-based resin layer that includes a second polyolefin-based resin having a melting point of less than 95°C, and a third polyolefin-based resin layer that includes a third polyolefin-based resin having a melting point of from 95°C to 200°C, wherein the first polyolefin-based resin layer, the second polyolefin-based resin layer, and the third polyolefin-based resin layer are disposed in this order, and the laminated film has an oxygen permeability of from 7,500 mL/m²·day·atm to 85,000 mL/²·day·atm.

The laminated film of the present disclosure, which has the above-described configuration, includes a low-melting-point polyolefin-based resin, and has an excellent gas permeability.

As described above, use of a low-density resin (e.g., a low-density polyolefin-based resin) in produce of a film is considered to be preferable from the standpoint of providing an excellent gas permeability.

However, there is a tendency that low-density resins have low melting points. In the case of trying to produce a film using a low-melting-point resin, there are problems in that, for example, production of a film is impossible due to the inability of the resin to maintain its shape, and it has been difficult to produce a film that retains product quality when a low-melting-point resin is used.

The above-described configuration enables the laminated film of the present disclosure to become free of stickiness and to maintain its film shape. Further, the laminated film of the present disclosure has an excellent gas permeability as well as an excellent heat sealability.

In the laminated film of the present disclosure, the first polyolefin-based resin layer, the second polyolefin-based resin layer, and the third polyolefin-based resin layer are disposed in layers in this order.

In other words, the laminated film is formed by allowing the second polyolefin-based resin layer, which have a low melting point (i.e. low density), to be sandwiched between the first polyolefin-based resin layer and the third polyolefin-based resin layer, which have a high melting point (i.e. high density).

The laminated film of the present disclosure has an excellent gas permeability due to the laminated film including the second polyolefin-based resin layer having a low melting point (i.e. low density). In addition, the laminated film does not have stickiness and is capable of maintaining its film shape, due to the second polyolefin-based resin layer being sandwiched between the first polyolefin-based resin layer and the third polyolefin-based resin layer, which have a high melting point (i.e. high density).

### -Gas Permeability-

### (Oxygen Permeability)

The laminated film of the present disclosure has an oxygen permeability of from 7,500 mL/m²·day·atm to 85,000 mL/m²·day·atm.

From the standpoint of long-term preservation of fruits and vegetables, flowers, microorganisms, and the like, the oxygen permeability of the laminated film of the present disclosure is preferably from 7,500 mL/m²·day·atm to 80,000 mL/m²·day·atm, and more preferably from 10,000 mL/m²·day·atm to 50,000 mL/m²·day·atm.

### (Carbon Dioxide Permeability)

The carbon dioxide permeability of the laminated film of the present disclosure is not particularly limited.

From the standpoint of long-term preservation of fruits and vegetables, flowers, microorganisms, and the like, the carbon dioxide permeability of the laminated film of the present disclosure is preferably from 8,000 mL/m²·day·atm to 250,000 mL/m²·day·atm, more preferably from 35,000 mL/m²·day·atm to 250,000 mL/m²·day·atm, still more preferably from 50,000 mL/m²·day·atm to 230,000 mL/m²·day·atm, particularly preferably from 50,000 mL/m²·day·atm to 200,000 mL/m²·day·atm, even more preferably from 50,000 mL/m²·day·atm to 150,000 mL/m²·day·atm.

### (Hydrogen Permeability)

The hydrogen permeability of the laminated film of the present disclosure is not particularly limited.

From the standpoint of suitability for use in hydrogen battery-related members such as hydrogen separation membranes and hydrogen permeable membranes, the hydrogen permeability of the laminated film of the present disclosure is preferably from 10,000 mL/m²·day·atm to 300,000 mL/m²·day·atm, more preferably from 50,000 mL/m²·day·atm to 230,000 mL/m²·day·atm, still more preferably from 50,000 mL/m²·day·atm to 200,000 mL/m²·day·atm, particularly preferably from 50,000 mL/m²·day·atm to 150,000 mL/m²·day·atm.

It is preferable that the laminated film of the present disclosure has an oxygen permeability of from 7,500 mL/m²·day·atm to 85,000 mL/m²·day·atm, a carbon dioxide permeability of from 8,000 mL/m²·day·atm to 250,000 mL/m²·day·atm, and a hydrogen permeability of from 10,000 mL/m²·day·atm to 300,000 mL/m²·day·atm.

The oxygen permeability (unit: mL/m²·day·atm), carbon dioxide permeability (unit: mL/m²·day·atm), and hydrogen permeability (unit: mL/m²·day·atm) of the laminated film can be measured in accordance with JIS K7126-1 (Differential-Pressure Method), JIS K7126-2 (Equal-Pressure Method), or the like.

For example, the oxygen permeability (unit: mL/m²·day·atm), carbon dioxide permeability (unit: mL/m²·day·atm), and hydrogen permeability (unit: mL/m²·day·atm) of the laminated film can be measured can be measured under the following conditions.

Measurement device: a gas permeability analyzer using the equal-pressure method (GTR-10XFKS, manufactured by GTR TEC Corporation)
Test piece: 50 mm diameter
Detector: gas chromatography type
Test gas: each gas species (carbon dioxide, oxygen, or hydrogen)
Measurement temperature: 23°C
Permeation area: 35 mm diameter

### <First Polyolefin-based Resin Layer>

The first polyolefin-based resin layer includes a first polyolefin-based resin having a melting point of from 95°C to 200°C.

The laminated film of the present disclosure is capable of maintaining its film shape, due to the laminated film including the first polyolefin-based resin layer. In addition, stickiness can be reduced.

The laminated film of the present disclosure exhibits an excellent heat sealability, due to the laminated film including the first polyolefin-based resin layer and the below-described third polyolefin-based resin layer.

The first polyolefin-based resin layer has a thickness of preferably from 1 µm to 20 µm, more preferably from 2 µm to 15 µm, still more preferably from 3 µm to 12 µm.

This provides an excellent heat-seal strength in the case of heat-sealing.

In the present disclosure, the thickness of the resin layer is measured using a contact-type thickness gauge (MODEL H, manufactured by PEACOCK).

The first polyolefin-based resin has a melting point of from 95°C to 200°C. This allows the laminated film of the present disclosure to maintain its film shape. In addition, stickiness can be reduced.

From this standpoint, the melting point of the first polyolefin-based resin is preferably from 95°C to 200°C, more preferably from 100°C to 190°C, still more preferably from 110°C to 180°C.

The first polyolefin-based resin layer preferably has a density of from 900 kg/m³ to 980 kg/m³ as measured in accordance with JIS K7112 (1999).

A density of the first polyolefin-based resin layer of 900 kg/m³ or more enables the resulting laminated film to maintain its film shape.

A density of the first polyolefin-based resin layer of less than 980 kg/m³ impartis an excellent heat sealability to the resulting laminated film.

From the foreging standpoints, the density of the first polyolefin-based resin layer as measured in accordance with JIS K7112 (1999) is more preferably from 905 kg/m³ to 950 kg/m³, and still more preferably from 910 kg/m³ to 930 kg/m³.

The first polyolefin-based resin is not particularly limited as long as the melting point of the first polyolefin-based resin is in the above-described range.

Examples of the first polyolefin-based resin include an ethylene homopolymer, a propylene homopolymer, and an *α*-olefin copolymer.

Examples of the ethylene homopolymer include low-density polyethylene (LDPE), linear low-density polyethylene (LLDPE), and high-density polyethylene (HDPE).

Examples of the *α*-olefin copolymer include a copolymer of two or more monomers selected from the group consisting of ethylene, propylene, 1-butene, 1-hexene, and 1-octene.

Examples of an ethylene-*α*-olefin copolymer include a copolymer of ethylene and one or more monomers selected from the group consisting of propylene, 1-butene, 1-hexene, and 1-octene.

The first polyolefin-based resin may be used singly, or in combination of two or more thereof.

The first polyolefin-based resin may include at least one selected from the group consisting of an unsaturated carboxylic acid-modified ethylene-*α*-olefin copolymer, an ethylene-unsaturated carboxylic acid-based copolymer, an ethylene-vinyl acetate copolymer, a silane-modified ethylene-vinyl acetate copolymer, an ionomer of an unsaturated carboxylic acid-modified ethylene-*α*-olefin copolymer, and an ionomer of an ethylene-unsaturated carboxylic acid-based copolymer. The first polyolefin-based resin preferably includes an ethylene-unsaturated carboxylic acid-based copolymer or an ionomer thereof.

### (Unsaturated Carboxylic Acid-Modified Ethylene-α-Olefin Copolymer)

An ethylene-*α*-olefin copolymer used in the unsaturated carboxylic acid-modified ethylene-*α*-olefin copolymer may be, for example, a copolymer of ethylene and an *α*-olefin having from 3 to 20 carbon atoms.

Examples of the *α*-olefin having from 3 to 20 carbon atoms include propylene, 1-butene, 1-hexene, 1-octene, 1-decene, 1-dodecene, 1-tetradecene, 1-hexadecene, 1-octadecene, and 1-eicosene.

Thereamong, from the standpoint of adhesion strength, the *α*-olefin having from 3 to 20 carbon atoms is preferably propylene, 1-butene, 1-hexene, or 1-octene, and more preferably 1-butene, 1-hexene, or 1-octene.

The molar ratio of ethylene to the *α*-olefin in the ethylene-*α*-olefin copolymer is preferably in a range of from 45/55 to 95/5.

In the present disclosure, examples of an unsaturated carboxylic acid used for the modification include unsaturated carboxylic acids and derivatives thereof.

An unsaturated carboxylic acid or a derivative thereof is used as the unsaturated carboxylic acid used for the modification, and examples thereof include unsaturated carboxylic acids such as maleic acid, fumaric acid, tetrahydrophthalic acid, itaconic acid, citraconic acid, crotonic acid, isocrotonic acid, nadic acid (registered trademark, endo-cis-bicyclo[2,2,1]hept-5-ene-2,3-dicarboxylic acid), acrylic acid and methacrylic acid, and derivatives thereof.

Examples of the derivative of an unsaturated carboxylic acid include an anhydride, imide, amide, or ester of the unsaturated carboxylic acid.

Specific examples of the derivative of an unsaturated carboxylic acid include maleimide, maleic anhydride, citraconic anhydride, monomethyl maleate, and glycidyl maleate.

Thereamong, as the unsaturated carboxylic acid used for the modification, an unsaturated carboxylic acid or an anhydride thereof is preferable, and maleic acid, maleic anhydride, nadic acid, and nadic anhydride are more preferable.

As the method for carrying out the modification with an unsaturated carboxylic acid is, for example, a method of carryiing out the modification with an unsaturated carboxylic acid by means of graft-reaction with the unsaturated carboxylic acid.

The method used for producing a modified product by means of graft copolymerization using the unsaturated carboxylic acid or a derivative thereof as a graft monomer may be a conventional known method.

For example, a melt modification method including melting an ethylene-*α*-olefin copolymer and adding a graft monomer thereto to cause graft copolymerization, or a solution modification method including dissolving an ethylene-*α*-olefin copolymer in a solvent and adding a graft monomer thereto to cause graft copolymerization, may be used.

When graft copolymerization is performed, the reaction is preferably carried out in the presence of a radical initiator from the standpoint of efficiency.

The content of the radical initiator may be in a range of from 0.001 parts by mass to 2 parts by mass with respect to 100 parts by mass of the ethylene-*α*-olefin copolymer, which is the base polymer.

Examples of the radical initiator include organic peroxides, such as dicumyl peroxide, di-*tert*-butyl peroxide, 2,5-dimethyl-2,5-di(*tert*-butylperoxy)hexine-3,2,5-dimethyl-2,5-di(*tert*-butylperoxy)hexane, and 1,4-bis(*tert*-butylperoxyisopropyl)benzene.

In the unsaturated carboxylic acid-modified ethylene-*α*-olefin copolymer in the present disclosure, the modification amount may be from 0.01% by mass to 10% by mass, preferably from 0.1% by mass to 5% by mass, and more preferably from 1% by mass to 5% by mass, with respect to the total amount of the unsaturated carboxylic acid-modified ethylene-*α*-olefin copolymer.

The unsaturated carboxylic acid-modified ethylene-*α*-olefin copolymer has a melt flow rate (MFR) in a range of preferably from 0.05 g/10 min to 200 g/10 min, more preferably from 0.1 g/10 min to 100 g/10 min, when measured in accordance with ASTM D1238 at a temperature of 190°C and a load of 2.16 kg.

Further, the unsaturated carboxylic acid-modified ethylene-*α*-olefin copolymer may have a degree of crystallinity of 30% or less as measured using X-ray.

### (Ethylene-Unsaturated Carboxylic Acid-based Copolymer)

The ethylene-unsaturated carboxylic acid-based copolymer is a copolymer that includes a structural unit derived from ethylene and a structural unit derived from an unsaturated carboxylic acid.

The ethylene-unsaturated carboxylic acid-based copolymer in the present disclosure may be a copolymer of ethylene and an unsaturated carboxylic acid, a copolymer of ethylene, an unsaturated carboxylic acid, and other monomer(s), or a polymer obtained by modification of an ethylene polymer with an unsaturated carboxylic acid.

Examples of the ethylene-unsaturated carboxylic acid-based copolymer include: ethylene-unsaturated carboxylic acid binary copolymers and ionomers thereof; ethylene-unsaturated carboxylic acid-unsaturated carboxylic acid ester ternary copolymers and ionomers thereof; and ethylene-unsaturated carboxylic acid ester binary copolymers. Among them, ethylene-unsaturated carboxylic acid-unsaturated carboxylic acid ester ternary copolymers and ionomers thereof are preferable from the standpoint of interlayer adhesion strength and heat-seal strength.

The ethylene-unsaturated carboxylic acid-based copolymer can be obtained by, for example, allowing ethylene and an unsaturated carboxylic acid to undergo a radical polymerization reaction at a high-temperature and high-pressure in the presence of an organic peroxide or oxygen.

Examples of the unsaturated carboxylic acid include ethyl acrylate, methacrylic acid, methyl methacrylate, vinyl acetate, and vinyl chloride.

Thereamong, from the standpoint of providing an excellent heat resistance and allowing high-temperature processing, the unsaturated carboxylic acid is preferably at least one selected from the group consisting of ethyl acrylate, methacrylic acid, methyl methacrylate, and vinyl acetate, and more preferably at least one selected from the group consisting of ethyl acrylate, methacrylic acid, and methyl methacrylate.

As the ethylene-unsaturated carboxylic acid-based copolymer, a copolymer of ethylene and ethyl acrylate (hereinafter also referred to as "EEA") can be used.

In the EEA, the content of ethyl acrylate may be from 10% by mass to 40% by mass, and preferably from 15% by mass to 30% by mass, from the standpoint of moldability and interlayer adhesion strength.

Further, the melt flow rate thereof (according to ASTM D1238; measured at a temperature of 190°C and a load of 2.16 kg) may be from 5 g/10 min to 50 g/10 min, and preferably from 10 g/10 min to 30 g/10 min, from the standpoint of moldability and interlayer adhesion strength, .

As the ethylene-unsaturated carboxylic acid-based copolymer, a copolymer of ethylene and methacrylic acid (hereinafter also referred to as "EMAA") can be used.

In the ethylene-methacrylic acid copolymer, the content of methacrylic acid may be from 10% by mass to 40% by mass, and preferably from 3% by mass to 10% by mass, from the standpoint of moldability and interlayer adhesion strength.

Further, the melt flow rate thereof (according to ASTM D1238; measured at a temperature of 190°C and a load of 2.16 kg) may be from 5 g/10 min to 100 g/10 min, and preferably from 10 g/10 min to 80 g/10 min, from the standpoint of moldability and interlayer adhesion strength, .

Examples of commercial products thereof include NUCREL (registered trademark) manufactured by Dow-Mitsui Polychemicals Co., Ltd.

(Ethylene-Vinyl Acetate Copolymer and Silane-Modified Ethylene-Vinyl Acetate Copolymer)

The ethylene-vinyl acetate copolymer (hereinafter also referred to as "EVA") is a copolymer of ethylene and vinyl acetate.

The silane-modified ethylene-vinyl acetate copolymer (hereinafter also referred to as "silane-modified EVA") is a compound obtained by modification of EVA.

From the standpoint of adhesion strength, the content of vinyl acetate in the EVA is preferably from 10% by mass to 50% by mass.

The EVA has a density in a range of preferably from 930 kg/m³ to 980 kg/m³.

The EVA has a melt flow rate (MFR) of preferably from 0.8 g/10 min to 30 g/10 min at a temperature of 190°C and a load of 2.16 kg.

The ethylene-vinyl acetate copolymer to be used may be an ethylene-vinyl acetate copolymer prepared by a conventional known method, or a commercially available product.

A silane compound used for modifying the EVA is preferably an organosilicon compound having at least one unsaturated hydrocarbon group and at least one alkoxy group in a molecule thereof.

Examples of the unsaturated hydrocarbon group include a vinyl group, an allyl group, and a (meth)acryl group, and examples of the alkoxy group include a methoxy group, an ethoxy group, and a butoxy group.

The silane compound is preferably, for example, vinyltrimethoxysilane or vinyltriethoxysilane, in which the unsaturated hydrocarbon group is a vinyl group and the alkoxy groups are methoxy groups or ethoxy groups.

In the silane-modified EVA, the content of the silane compound is preferably from 0.01 parts by mass to 5.0 parts by mass with respect to 100 parts by mass of EVA.

The silane-modified EVA in the present disclosure may be a compound obtained by graft modification of EVA with a silane compound.

For example, the silane-modified EVA can be obtained by adding a silane compound and a radical generator to EVA in an extruder, performing melt-kneading at a temperature and for a time that are not lower than the thermal decomposition onset temperature of the radical generator, and then performing pelletization.

The radical generator is a compound that is decomposed by heating to generate a free radical, and is used as a reaction initiator that chemically binds a silane compound to EVA. A radical generator that has a half-life of 3 minutes or less at a temperature of from the melting point of the EVA to 150°C is particularly preferable.

Examples of the radical generator include peroxides, such as benzoyl peroxide, lauroyl peroxide, *t*-butyl peracetate, *t*-butylperoxy-2-ethylhexanoate, *t-*butylperoxyisobutyrate, and dicumyl peroxide.

The content of the radical generator may be adjusted, as appropriate, in accordance with the type of the EVA and the amount of the silane compound used.

For example, the content of the radical generator is preferably from 0.01 parts by mass to 5.0 parts by mass with respect to 100 parts by mass of EVA.

Further, a reaction inhibitor such as mercaptan may be added, in order to reduce polymerization between the silane compound molecules.

Among the above-described polymers, the first polyolefin-based resin is preferably at least one selected from the group consisting of an ethylene homopolymer, a propylene homopolymer, and an ethylene-*α*-olefin copolymer that is a copolymer of ethylene and one or more *α*-olefins having from 3 to 8 carbon atoms, and more preferably at least one selected from the group consisting of a low-density polyethylene and a linear low-density polyethylene.

### <Second Polyolefin-based Resin Layer>

The second polyolefin-based resin layer includes a second polyolefin-based resin having a melting point of less than 95°C.

The laminated film of the present disclosure has an improved gas permeability due to including the second polyolefin-based resin layer in the laminated film.

Further, the laminated film of the present disclosure has an excellent resistance to low temperature (hereinafter also referred to as "low-temperature characteristics") due to inclusion of the second polyolefin-based resin layer in the laminated film. For example, in a case in which the laminated film of the present disclosure is used for refrigerated or frozen storage of contents such as cells, cracking, delamination, and the like at low temperatures are reduced.

The second polyolefin-based resin layer has a thickness of preferably from 5 µm to 100 µm, more preferably from 7 µm to 50 µm, and still more preferably from 9 µm to 30 µm.

In the present disclosure, the thickness of a resin layer is measured using a contact-type thickness gauge (MODEL H, manufactured by PEACOCK).

The second polyolefin-based resin has a melting point of less than 95°C, which imparts improved gas permeability to the laminated film of the present disclosure.

From this standpoint, the melting point of the second polyolefin-based resin is preferably 90°C or less, and more preferably 85°C or less.

The second polyolefin-based resin layer preferably has a density of from 800 kg/m³ to less than 900 kg/m³ as measured in accordance with JIS K7112 (1999).

A density of the second polyolefin-based resin layer of 800 kg/m³ or more enables the resulting laminated film to maintain its film shape.

A density of the second polyolefin-based resin layer of less than 900 kg/m³ imparts an excellent gas permeability to the resulting laminated film.

From the foregoing standpoints, the density of the second polyolefin-based resin layer as measured in accordance with JIS K7112 (1999) is more preferably from 820 kg/m³ to 890 kg/m³, still more preferably from 830 kg/m³ to 890 kg/m³, and further more preferably from 850 kg/m³ to 888 kg/m³.

The second polyolefin-based resin is not particularly limited as long as the melting point thereof is in the above-described range.

Similarly to the case of the above-described first polyolefin-based resin, examples of the second polyolefin-based resin include an ethylene homopolymer, a propylene homopolymer, and an *α*-olefin copolymer.

The specifics of the ethylene homopolymer, the propylene homopolymer, and the *α-*olefin copolymer for the second polyolefin-based resin, such as specific examples, specific aspects, and preferable aspects, are the same as the specifics of the ethylene homopolymer, the propylene homopolymer, and the *α*-olefin copolymer, such as specific examples, specific aspects, and preferable aspects, described in the explanation of the first polyolefin-based resin.

The second polyolefin-based resin is preferably at least one selected from the group consisting of an ethylene homopolymer and an ethylene-*α*-olefin copolymer that is a copolymer of ethylene and one or more *α*-olefins having from 3 to 8 carbon atoms, and is more preferably at least one selected from the group consisting of an ethylene homopolymer and an ethylene-1-butene copolymer that is a copolymer of ethylene and 1-butene.

The second polyolefin-based resin is preferably an ethylene-*α*-olefin copolymer having a melting point of less than 90°C.

### <Third Polyolefin-based Resin Layer>

The third polyolefin-based resin layer includes a third polyolefin-based resin having a melting point of from 95°C to 200°C.

The laminated film of the present disclosure is capable of maintaining its film shape, due to inclusion of the third polyolefin-based resin layerin the laminated film. In addition, stickiness can be reduced.

The third polyolefin-based resin in the present disclosure and the first polyolefin-based resin in the present disclosure may be the same resin or different resins. It is preferable that the third polyolefin-based resin and the first polyolefin-based resin are the same resin.

The third polyolefin-based resin layer has a thickness of preferably from 1 µm to 20 µm, more preferably from 2 µm to 15 µm, and still more preferably from 3 µm to 12 µm.

Such a thickness imparts an excellent heat-seal strength in the case of heat-sealing.

In the present disclosure, the thickness of a resin layer is measured using a contact-type thickness gauge (MODEL H, manufactured by PEACOCK).

The third polyolefin-based resin has a melting point of from 95°C to 200°C. Due to this feature, the laminated film of the present disclosure is capable of maintaining its film shape. In addition, stickiness can be reduced.

From these standpoints, the melting point of the third polyolefin-based resin is preferably from 95°C to 200°C, more preferably from 100°C to 190°C, and still more preferably from 110°C to 180°C.

The third polyolefin-based resin layer preferably has a density of from 900 kg/m³ to 980 kg/m³ as measured in accordance with JIS K7112 (1999).

A density of the third polyolefin-based resin layer of 900 kg/m³ or more enables the resulting laminated film to maintain its film shape.

A density of the third polyolefin-based resin layer of less than 980 kg/m³ imparts an excellent heat sealability to the resulting laminated film.

From these standpoints, the density of the third polyolefin-based resin layer as measured in accordance with JIS K7112 (1999) is more preferably from 905 kg/m³ to 950 kg/m³, and still more preferably from 910 kg/m³ to 930 kg/m³.

The third polyolefin-based resin is not particularly limited as long as the melting point is within the above-described range.

Similarly to the above-described first polyolefin-based resin, examples of the third polyolefin-based resin include an ethylene homopolymer, a propylene homopolymer, and an *α*-olefin copolymer.

The specifics of the ethylene homopolymer, the propylene homopolymer, and the *α-*olefin copolymer for the third polyolefin-based resin, such as specific examples, specific aspects, and preferable aspects, are the same as the specifics of the ethylene homopolymer, the propylene homopolymer, and the *α*-olefin copolymer, such as specific examples, specific aspects, and preferable aspects, described in the explanation of the first polyolefin-based resin.

Among the above-described polymers, the third polyolefin-based resin is preferably at least one selected from the group consisting of an ethylene homopolymer, a propylene homopolymer, and an ethylene-*α*-olefin copolymer that is a copolymer of ethylene and one or more *α*-olefins having from 3 to 8 carbon atoms, and more preferably at least one selected from the group consisting of a low-density polyethylene and a linear low-density polyethylene.

It is more preferable that each of the first polyolefin-based resin layer and the third polyolefin-based resin layer has a density of from 900 kg/m³ to 980 kg/m³ as measured in accordance with JIS K7112.

In other words, it is more preferable that the first polyolefin-based resin layer and the third polyolefin-based resin layer both have a density of from 900 kg/m³ to 980 kg/m³ as measured in accordance with JIS K7112.

It is preferable that each of the first polyolefin-based resin and the third polyolefin-based resin is independently at least one selected from the group consisting of an ethylene homopolymer, a propylene homopolymer, and an ethylene-*α*-olefin copolymer that is a copolymer of ethylene and one or more *α*-olefins having from 3 to 8 carbon atoms.

It is preferable that at least one of the first polyolefin-based resin layer or the third polyolefin-based resin layer has a thickness of from 5 µm to 100 µm, and it is more preferable that both of the first polyolefin-based resin layer and the third polyolefin-based resin layer have a thickness of from 5 µm to 100 µm.

The thickness ratio of the second polyolefin-based resin layer to the total thickness of the first polyolefin-based resin layer and the third polyolefin-based resin layer (second/first and third) is preferably from 0.5 to 20.0.

When the thickness ratio (second/first and third) is in this range, the gas permeability and the capability of maintaining the film shape are adjusted in a well-balanced manner in the resulting laminated film.

From this standpoint, the thickness ratio (second/first and third) is more preferably from 0.7 to 10.0, and still more preferably from 1.0 to 5.0.

The laminated film of the present disclosure has a total thickness of preferably from 15 µm to less than 150 µm, more preferably from 20 µm to 100 µm, and still more preferably from 25 µm to 60 µm.

The first to the third polyolefin-based resins in the present disclosure, may be the same resin or different resins. The first to the third polyolefin-based resins are preferably the same resin.

When the first to the third polyolefin-based resins in the present disclosure are the same resin, excellent recyclability is achieved.

### [Method of Producing Laminated Film]

The method used for producing the laminated film of the present disclosure is not particularly limited. For example, a method is preferable in which the respective constituent layers of the laminated film of the present disclosure are adhered to each other due to intermingling thereof at or around the interfaces, thereby forming the laminated film.

Examples of such a method include a coextrusion method including forming a laminated body of molten resins, and a heat fusion method including heat-fusing pre-formed resin laminated films.

Of these methods, the coextrusion method including forming a laminated body of molten resins is more preferable from the viewpoint that this method is capable of forming a laminated film having high interlayer adhesion strength between layers and being less likely to experience delamination between layers.

The laminated film of the present disclosure is preferably formed by a coextrusion blown film molding method, a coextrusion cast film molding method, or a coextrusion lamination molding method.

It is preferable to perform, for example, a surface treatment on a specific layer before layering the respective layers of the laminated film of the present disclosure.

By performing the surface treatment, more improved interlayer adhesion strength can be obtained.
Examples of the surface treatment include corona treatment and ozone treatment.
Corona treatment can be performed using, for example, CORONA MASTER (e.g., PS-10S manufactured by Shinko Electric & Instrumentation Co., Ltd.).

The ozone treatment can be performed using, for example, a UV ozone cleaner (e.g., UV42 manufactured by Nippon Laser & Electronics).

### [Use of Laminated Film]

The laminated film of the present disclosure can widely be used as, for example, a tape, an adhesive tape, a masking tape, a masking laminated film, a temporarily attaching laminated film, a freshness-maintaining packaging laminated film, a plastic envelope, an easy-open packaging bag, an automatic packaging laminated film, a shopping bag, a standing bag, a liquid laminated film container, a transparent packaging box, a building material, a laminated film for bonding together, an agricultural laminated film, a food packaging material, a fruit packaging material, a flower packaging material, an electronic component packaging material, a machine parts packaging material, a grain packaging material, a packaging material for a fishery product such as seafood, a medical laminated film, a medical tape, a plant cell culture pack, a seed/seedling storage/growth pack, an animal cell culture pack, a carbon dioxide separation membrane, a hydrogen separation membrane, or an oxygen enrichment membrane.

### <<Container>>

The container of the present disclosure is formed of the laminated film of the present disclosure and is heat-sealed.

The container is not particularly limited in terms of its shape and the like as long as the container is capable of accommodating therein an item to be contained. The container may have, for example, a bag shape.

The container of the present disclosure may be, for example, a container for storing a food, a fruit/vegetable, a flower/ornamental plant, a seed/seedling, a microorganism, a cell, or the like.

Among the foregoing, the container of the present disclosure is preferably a cell culture container.

### «Cell Culture Container»

The cell culture container of the present disclosure includes the laminated film of the present disclosure.

The laminated film of the present disclosure includes a low-melting-point polyolefin-based resin and has an excellent gas permeability. Therefore, the cell culture container that includes the laminated film of the present disclosure also has an excellent gas permeability. This makes it possible to maintain the oxygen concentration inside the container in a favorable state even during cell culture.

In conventional processes, the oxygen concentration inside a container is maintained by, for example, a method such as periodically injecting oxygen into inside the container during cell culture. We surmise that the cell culture container of the present disclosure does not necessitate periodic injection of oxygen into inside the container, due to the cell culture container of the present disclosure having an excellent gas permeability.

The cell culture container may be, for example, a cell culture pack.

The cell culture container of the present disclosure may be formed of the laminated film of the present disclosure, or may include the laminated film of the present disclosure at a part of the cell culture container.

The cell culture container of the present disclosure preferably includes the laminated film of the present disclosure at a bottom portion of the container.

The cell culture container of the present disclosure is not particularly limited in terms of shape.

In the cell culture container of the present disclosure, the edge portions of the laminated film are preferably heat-sealed to form a bag.

The cell culture container of the present disclosure preferably has a shape that allows hermetical sealing.

For example, a culture container in which the edge portions of the laminated film are heat-sealed to form a bag may include a port for taking a sample in and out of the bag.

The cell culture container of the present disclosure preferably has a medical device Sterility Assurance Level (SAL) as measured in accordance with BS EN 556-1:2001 of 10⁻⁶ or less.

When the SAL is in this range, the inside of the container is considered to be in a state of being free from viable microorganisms, which indicates excellent sterility.

### «Cell Culture Method»

The cell culture method of the present disclosure includes a step of enclosing a cell in the cell culture container of the present disclosure, and hermetically sealing the cell culture container.

As described above, we surmise that the cell culture container of the present disclosure does not necessitate periodic injection of oxygen into inside the container, due to the cell culture container exhibiting an excellent gas permeability. Accordingly, it is conceivable that once the step of enclosing a cell in the cell culture container of the present disclosure and hermetically sealing the cell culture container is completed, subsequent cultivation of the cell can be carried out without periodically injecting oxygen into inside the container.

### <<Method of Producing Cell Culture Container>>

The method of producing a cell culture container according to the present disclosure includes a film-forming step of coextruding a third polyolefin-based resin layer including a first polyolefin-based resin having a melting point of from 95°C to 200°C, a second polyolefin-based resin layer including a second polyolefin-based resin having a melting point of less than 95°C, and a third polyolefin-based resin layer including a third polyolefin-based resin having a melting point of from 95°C to 200°C, to obtain a laminated film.

In the film-forming step of the method of producing a cell culture container according to the present disclosure, the laminated film is preferably obtained using, for example, a blown film molding machine such as a T-die extrusion molding machine or an extrusion-lamination molding machine.

The blown film molding conditions may be selected as appropriate.

The molding conditions may be, for example, the following conditions.

### -Molding Conditions-

Molding machine: three-layer blown film molding machine (manufactured by Alpine: three 50-mm diameter extruders)
Die: 225 mmcp (diameter), 3.5 mm (lip width)
Air ring: 2-gap type
Molding temperature: 200°C
Extrusion rate: (first layer (outermost layer): 5.3 kg/hr, second layer (interlayer): 10.2/hr, third layer (innermost layer): 5.3 kg/hr)
Take-up speed: 10 m/min

The method of producing a cell culture container according to the present disclosure preferably further includes a bag-forming step of heat-sealing edge portions of the obtained laminated film, to form a bag.

The heat-sealing temperature is preferably from 100°C to 200°C, more preferably from 110°C to 170°C, and still more preferably from 120°C to 150°C.

Specific aspects of the heat-sealing are not particularly limited. The heat-sealing may be carried out, for example, according to the following aspect.

Specifically, the aspect includes: preparing two sheests of the laminated film; arranging faces of the laminated film sheets to face each other; heat-sealing edge portions thereof, with a seal bar width of 5 mm and at a heat-sealing temperature of 140°C and a pressure of 0.2 MPa for a sealing time of 1 second; and thereafter allowing the resultant to cool.

The method of producing a cell culture container according to the present disclosure preferably further includes a step of irradiating the obtained bag with a gamma ray.

Irradiation with a gamma ray enables sterilization of the inside of the bag, which improves the quality as a cell culture container.

The absorbed dose of the gamma ray is preferably from 5 kGy to 50 kGy, and the irradiation is preferably performed until the medical device Sterility Assurance Level (SAL) of the cell culture container becomes 10⁻⁶ or less.

### EXAMPLES

The present disclosure is more specifically described below by way of examples. However, the present disclosure is not limited to the examples described below as far ast the gist of the present disclosure is retained.

The following materials were used in the examples.

### [Polyolefin]

LLDPE: C6 linear low-density polyethylene copolymer (EVOLUE SP2020 manufactured by Prime Polymer Co., Ltd., having a melting point of 116°C and a density of 916 kg/m³)
PO1: linear low-density polyethylene copolymer (AFFINITY (registered trademark) VP 8770G1 manufactured by Dow Chemical Japan, Ltd., having a melting point of 82°C and a density of 886 kg/m³)
PO2: ethylene-butene copolymer (TAFMER (registered trademark) DF640 manufactured by Mitsui Chemicals, Inc., having a melting point of 50°C or less and a density of 864 kg/m³)

### [Laminated Film]

### <Example 1>

Using the polyolefin indicated in Table 1, a three-layer laminated film having a thickness of 30 µm and a width of 600 mm was produced using a blown film molding method under the below-described molding conditions. In the laminated film, the first polyolefin-based resin layer (hereinafter also referred to as "first PO layer"), the second polyolefin-based resin layer (hereinafter also referred to as "second PO layer"), and the third polyolefin-based resin layer (hereinafter also referred to as "third PO layer") had a thickness ratio (the first PO layer: the second PO layer: the third PO layer) indicated in Table 1.

### -Molding Conditions-

Molding machine: a three-layer blown film molding machine (manufactured by Alpine: three 50-mm diameter extruders)
Die: 225 mmcp (diameter), 3.5 mm (lip width)
Air ring: 2-gap type
Molding temperature: 200°C
Extrusion rate: (first PO layer (outermost layer): 5.3 kg/hr, second PO layer (interlayer): 10.2/hr, third PO layer (innermost layer): 5.3 kg/hr)
Take-up speed: 10 m/min

### <Example 2>

A three-layer laminated film was produced in the same manner as that in Example 1, except that the extrusion rates of the first PO layer, the second PO layer, and the third PO layer were changed to 4.3 kg/hr, 12.2 kg/hr, and 4.3 kg/hr, respectively.

In the laminated film, the thickness ratio (the first PO layer: the second PO layer: the third PO layer) was as indicated in Table 1.

### <Example 3>

A three-layer laminated film was produced in the same manner as that in Example 1, except that the extrusion rates of the first PO layer, the second PO layer, and the third PO layer were changed to 3.1 kg/hr, 14.6 kg/hr, and 3.1 kg/hr, respectively.

In the laminated film, the thickness ratio (the first PO layer: the second PO layer: the third PO layer) was as indicated in Table 1.

### <Example 4>

Using the polyolefin indicated in Table 1, a three-layer laminated film having a thickness of 30 µm and a width of 720 mm was produced using a cast film molding method under the below-described molding conditions. In the laminated film, the thickness ratio of the first PO layer, the second PO layer, and the third PO layer (the first PO layer: the second PO layer: the third PO layer) was as indicated in Table 1.

### -Molding Conditions-

Molding machine: a 50-mm diameter multilayer cast molding machine equipped with a T-die (manufactured by Sumitomo Heavy Industries Modern, Ltd.: a 50-mm diameter extruder and two 40-mm diameter extruders)
Cylinder temperature: 200°C
Chill roll temperature: 18°C
Take-up speed: 30 m/min

### <Example 5>

The laminated film obtained in Example 3 was cut into a rectangular shape, an introduction port and a discharge port were provided on one side of the rectangular laminated film, and the peripheral edge portions of the rectangular laminated film were heat-sealed at 140°C for a width of 5 mm, whereby a cell culture bag having a capacity of 1,000 mL was produced.

The cell culture bag obtained was irradiated with a gamma ray, and it was confirmed that the medical device Sterility Assurance Level (SAL) as measured in accordance with BS EN 556-1:2001 was 10⁻⁶ or less. The sterilized cell culture bag had a favorable outer appearance, and discoloration or the like was not observed.

### <Comparative Example 1>

A single-layer film having only the first PO layer was produced in the same manner as that in Example 1, except that the polyolefin indicated in Table 1 was used, and that the second PO layer and the third PO layer were not provided.

### <Comparative Example 2>

The same procedures as those in Example 1 were carried out, except that the polyolefin indicated in Table 1 was used, and that the first PO layer and the third PO layer were not arranged, for the purpose of producing a single-layer film having only the second PO layer

However, the product obtained was sticky, and could not be formed into a film. Thus, a single-layer film having only the second PO layer could not be produced.

### [Evaluation]

### (Gas Permeability)

The oxygen permeability (unit: mL/m²·day·atm), carbon dioxide permeability (unit: mL/m²·day·atm) and hydrogen permeability (unit: mL/m²·day·atm) of a laminated film are measured under the conditions described below.

A laminated film is considered to have an excellent gas permeability if the laminated film has a carbon dioxide permeability of 70,000 mL/m²·day·atm or more, an oxygen permeability of 20,000 mL/m²·day·atm or more, and a hydrogen permeability of 10,000 mL/m²·day·atm or more.

### Measuring device: a gas permeability analyzer using the equal-pressure method (GTR-10XFKS, manufactured by GTR TEC Corporation)

Test piece: 50 mm diameter
Detector: gas chromatography type
Test gas: each gas species (carbon dioxide, oxygen, or hydrogen)
Measurement temperature: 23°C
Transmission area: 35 mm diameter

### (Heat Sealability)

The heat sealability of a film was evaluated by the following method.

Two sheets of the film were prepared, and faces of the film sheets were allowed to face each other and heat-sealed with a seal bar width of 5 mm and at a heat-sealing temperature of 140°C and a pressure of 0.2 MPa for a sealing time of 1 second. Thereafter, the resultant was allowed to cool. Subsequently, a 15 mm-wide test piece was cut out from the sample obtained by the heat-sealing, and the peel strength was measured while the sheets were separated at the heat-sealed portion at a crosshead speed of 300 mm/min. Heat sealability was evaluated using the obtained value as the heat-seal strength.

### -Evaluation Criteria-

A: 5 N/15 mm or more
B: less than 5 N/15 mm

### (Low-Temperature Characteristics)

A test piece having a width of 210 mm and a length of 297 mm was cut out from each of the films obtained in the Examples and the Comparative Examples, and subjected to a bending test in which the film was bent 1,000 times to a bending angle of 440° in a 4°C atmosphere with a bending rate of 40 times/min, using a Gelbo flex tester manufactured by Tester Sangyo Co., Ltd. Thereafter, a bag was produced from the test piece, and the number of pinholes was measured using AGELESS SEAL CHECK manufactured by Mitsubishi Gas Chemical Co., Inc. Evaluation was carried out according to the following evaluation criteria.

### -Evaluation Criteria-

A: The number of pinholes was less than 50 pinholes/m².
B: The number of pinholes was 50 pinholes/m² or more.

**[Table 1]**

| | | | Example 1 | Example 2 | Example 3 | Example 4 | Comparative Example 1 | Comparative Example 2 |
|---|---|---|---|---|---|---|---|---|
| Laminated film | First polyolefin-based resin layer | Type | LLDPE | LLDPE | LLDPE | LLDPE | LLDPE | - |
| | | Thickness [µm] | 7.5 | 6 | 4.3 | 10 | 30 | - |
| | | Melting point [°C] | 116 | 116 | 116 | 116 | 116 | - |
| | | Density [kg/m³] | 916 | 916 | 916 | 916 | 916 | - |
| | Second polyolefin-based resin layer | Type | PO1 | PO1 | PO1 | PO2 | - | PO1 |
| | | Thickness [µm] | 15 | 18 | 21.4 | 10 | - | 30 |
| | | Melting point [°C] | 82 | 82 | 82 | <50 | - | 82 |
| | | Density [kg/m³] | 886 | 886 | 886 | 864 | - | 886 |
| | Thrid polyolefin-based resin layer | Type | LLDPE | LLDPE | LLDPE | LLDPE | - | - |
| | | Thickness [µm] | 7.5 | 6 | 4.3 | 10 | - | - |
| | | Melting point [°C] | 116 | 116 | 116 | 116 | - | - |
| | | Density [kg/m³] | 916 | 916 | 916 | 916 | - | - |
| | Thickness ratio | | 1:2:1 | 1:3:1 | 1:5:1 | 1:1:1 | single layer | single layer |
| | First PO layer: Second PO layer: Third PO layer | | | | | | | |
| | Oxygen permeability [mL/m²·day·atm] | | 16,000 | 21,000 | 23,000 | - | 6,000 | - |
| | Carbon dioxide permeability [mL/m²·day·atm] | | 56,000 | 79,000 | 81,000 | - | 33,000 | - |
| | Hydrogen permeability [mL/m²·day·atm] | | - | - | 71,000 | - | - | - |
| Evaluation | | Heat sealability | A | A | A | A | A | - |
| | | Low-temperature characteristics | A | A | A | A | B | - |

In the Examples, in which a laminated film that includes a first polyolefin-based resin layer including a first polyolefin-based resin having a melting point of from 95°C to 200°C, a second polyolefin-based resin layer including a second polyolefin-based resin having a melting point of less than 95°C, and a third polyolefin-based resin layer including a third polyolefin-based resin having a melting point of from 95°C to 200°C layered in this order was used, a low-melting-point polyolefin-based resin is included in the second PO layer, and the laminated film exhibited an excellent gas permeability, as shown in Table 1.

In contrast, in Comparative Example 1, which is a single-layer film not including a second polyolefin-based resin having a melting point of less than 95°C, did not include a low-melting-point polyolefin-based resin, and exhibited an inferior gas permeability.

In Comparative Example 2, in which production of a single-layer film was attempted using a second polyolefin-based resin having a melting point of less than 95°C, a single-layer film could not be produced.

The disclosure of Japanese Patent Application No. 2021-190482, filed November 24, 2021, is incorporated herein by reference in its entirety.

All documents, patent applications, and technical standards that are described in the present specification are incorporated herein by reference to the same extent as if each individual document, patent application, or technical standard is specifically and individually described to be incorporated by reference.

## Claims

1. A laminated film, comprising:
a first polyolefin-based resin layer comprising a first polyolefin-based resin having a melting point of from 95°C to 200°C;
a second polyolefin-based resin layer comprising a second polyolefin-based resin having a melting point of less than 95°C; and
a third polyolefin-based resin layer comprising a third polyolefin-based resin having a melting point of from 95°C to 200°C,
wherein the first polyolefin-based resin layer, the second polyolefin-based resin layer, and the third polyolefin-based resin layer are disposed in this order, and the laminated film has an oxygen permeability of from 7,500 mL/m²·day·atm to 85,000 mL/m²·day·atm.

2. The laminated film according to claim 1, wherein the laminated film has a total thickness of from 15 µm to less than 150 µm.

3. The laminated film according to claim 1, wherein the second polyolefin-based resin layer has a density of from 820 kg/m³ to 890 kg/m³ as measured in accordance with JIS K7112.

4. The laminated film according to claim 1, wherein the second polyolefin-based resin is an ethylene-*α*-olefin copolymer having a melting point of less than 90°C.

5. The laminated film according to claim 1, wherein each of the first polyolefin-based resin layer and the third polyolefin-based resin layer has a density of from 900 kg/m³ to 980 kg/m³ as measured in accordance with JIS K7112.

6. The laminated film according to claim 1, wherein the laminated film has a carbon dioxide permeability of from 35,000 mL/m²·day·atm to 250,000 mL/m²·day·atm.

7. The laminated film according to claim 1, wherein at least one of the first polyolefin-based resin layer or the third polyolefin-based resin layer has a thickness of from 5 µm to 100 µm.

8. The laminated film according to claim 1, wherein the second polyolefin-based resin layer has a thickness of from 5 µm to 100 µm.

9. The laminated film according to claim 1, wherein a ratio of a thickness of the second polyolefin-based resin layer to a total thickness of the first polyolefin-based resin layer and the third polyolefin-based resin is from 0.5 to 20.0.

10. The laminated film according to claim 1, wherein the laminated film is formed by a coextrusion blown film molding method, a coextrusion cast film molding method, or a coextrusion lamination molding method.

11. A container, formed from the laminated film according to any one of claims 1 to 10, wherein the container is heat-sealed.

12. A cell culture container, comprising the laminated film according to claim 1.

13. The cell culture container according to claim 12, comprising the laminated film at a bottom portion of the container.

14. The cell culture container according to claim 12, wherein edge portions of the laminated film are heat-sealed to form a bag.

15. The cell culture container according to claim 14, having a medical device Sterility Assurance Level (SAL) of 10⁻⁶ or less as measured in accordance with BS EN 556-1:2001.

16. A cell culture method, comprising a step of enclosing a cell in the cell culture container according to any one of claims 12 to 15 and hermetically sealing the cell culture container.

17. A method of producing a cell culture container, the method comprising a film-forming step of coextruding a third polyolefin-based resin layer comprising a first polyolefin-based resin having a melting point of from 95°C to 200°C, a second polyolefin-based resin layer comprising a second polyolefin-based resin having a melting point of less than 95°C, and a third polyolefin-based resin layer comprising a third polyolefin-based resin having a melting point of from 95°C to 200°C, to obtain a laminated film.

18. The method of producing a cell culture container according to claim 17, further comprising a bag-forming step of heat-sealing edge portions of the obtained laminated film, to form a bag.

19. The method of producing a cell culture container according to claim 18, further comprising a step of irradiating the obtained bag with a gamma ray.

20. The method of producing a cell culture container according to claim 17, wherein, in the film-forming step, the laminated film is obtained using a blown film molding machine.
